# EUROPÄISCHE PATENTSCHRIFT

(11) **EP 0 626 824 B1**
(45) Veröffentlichungstag und Bekanntmachung des Hinweises auf die Patenterteilung: **02.10.1996**
(21) Anmeldenummer: 93903169.6
(22) Anmeldetag: 13.02.1993
(51) Int. Cl.: A61B 17/00

(54) **VORRICHTUNG FÜR TRANSANALE RESEKTATEXTRAKTIONEN**
DEVICE FOR TRANSANAL RESECTATE EXTRACTION
DISPOSITIF POUR EXTRACTIONS TRANSANALES DE MATIERES DE RESECTION

(30) Priorität: 22.02.1992 DE 4205488
(43) Veröffentlichungstag der Anmeldung: 07.12.1994
(73) Patentinhaber: Forschungszentrum Karlsruhe GmbH, D-76133 Karlsruhe (DE)
(72) Erfinder: SCHÜLKEN, Heinrich, D-7513 Stutensee-Staffort (DE); SCHLIPF, Karl, D-7500 Karlsruhe 1 (DE)
(74) Vertreter: Rückert, Friedrich, Dr.
(86) Internationale Anmeldenummer: DE9300124
(87) Internationale Veröffentlichungsnummer: WO9316643

(56) Entgegenhaltungen:
- DE-A- 3 329 784
- DE-A- 3 823 604
- GB-A- 2 130 889

## Beschreibung

Die Erfindung betrifft eine Vorrichtung für transanale Resektatextraktionen gemäß dem Oberbegriff des ersten Patentanspruchs.

Neoplasien (Neubildung von Gewebe wie z. B. Tumore) und Angiodysplasien (Gefäßfehlbildungen) im Sigma können eine Resektion dieses Darmteils zwischen Colon descendens und Rektum erforderlich machen. Bei dieser Resektion wird der Darm vor und hinter dem Sigma per laparoskopiam mit Hilfe von durch die Bauchdecke eingeführten optischen und chirurgischen Instrumenten durchtrennt. Bevor das Colon descendens und das Rektum miteinander verbunden werden können, muß das Resektat entfernt werden. Die Extraktion des Resektats erfolgt durch den Anus.

Zur Extraktion des Resektats wird üblicherweise eine Rektoskop-Hülse transanorektal eingeführt und das proximale (dem Operateur zugewandte) Ende mit einem Anschlußstück verschlossen. Rektoskop-Hülsen mit einem äußeren Durchmesser von 40 mm und verschiedener Länge sind kommerziell erhältlich. Die bekannten Rektoskop-Hülsen sind an ihrem distalen (dem Operateur abgewandten) Ende angeschrägt. Sie werden mit Hilfe eines sogenannten Mandrins (eines Stabes mit einer Verdickung in Form eines Konus oder eines abgerundeten Zylinders am distalen Ende, deren Durchmesser etwas kleiner ist als der Innendurchmesser der Rektoskop-Hülse) eingeführt. Das Anschlußstück der Rektoskop-Hülse enthält einen Rohrstutzen, durch den Kohlendioxid eingeleitet werden kann, um den Darm aufzuweiten. Durch eine Öffnung im Anschlußstück wird eine Faßzange durch die Rektoskop-Hülse in das Rektum vorgeschoben. Der Laparoskopiker übergibt das Resektat an die Faßzange, mit deren Hilfe es durch die Rektoskop-Hülse transanal entfernt wird.

In einer Figur der DE 33 29 784 C2 ist eine solche Rektoskop-Hülse mit Anschlußstück dargestellt. Weiterhin ist eine Faßzange angedeutet, die durch das Anschlußstück in die Rektoskop-Hülse eingeschoben ist.

Beim transanorektalen Einschieben der Faßzange in das Abdomen sind bei dem beschriebenen Vorgehen - abhängig von der Anatomie und Histologie des Darmteils, der nicht durch die Rektoskop-Hülse geschützt ist, von der Übung und Erfahrung des Chirurgen und von der Ausführung der Faßzange - Läsionen und/oder Perforationen nicht auszuschließen.

Aus der DE 38 23 604 A1 ist eine Einrichtung zur intrakavitären Strahlentherapie von bösartigen Mastdarmgeschwulsten bekannt.

Die bekannte Vorrichtung weist einen Tubus auf, der einer Rektoskop-Hülse entspricht, und einen Endostat, der aus einem Hohlrohr und einer keulenförmigen Verdickung besteht. Die keulenförmige Verdickung ist ausgehöhlt, so daß radioaktives Material an die Behandlungsstelle gebracht werden kann.

Ferner ist aus der DE 82 33 240 U1 ein Rektoskop bekannt, das einen kurzen, weiten Tubus und einen verdrehbaren Kupplungsring enthält. Durch den Tubus wird ein Mandrin geführt. Nach dem Entfernen des Mandrins wird ein Instrumententräger als Anschlußstück über einen bajonettartigen Verschluß auf den Tubus aufgesetzt. Der Verschluß wird durch das Eingreifen von Sperrkugeln in eine Ringnut bewirkt.

Aufgabe der Erfindung ist, eine verbesserte Vorrichtung vorzuschlagen, mit der ein Darmabschnitt transanal extrahiert werden kann und bei der die Gefahr von Läsionen oder Perforationen zumindest stark vermindert ist. Eine weitere Aufgabe besteht darin, die Vorrichtung so auszugestalten, daß eine schnelle und einfache Bedienung durch den Operateur möglich ist.

Die Aufgabe wird bei einer Vorrichtung der eingangs genannten Art erfindungsgemäß durch das im ersten Patentanspruch gekennzeichnete Merkmal gelöst. Die weiteren Ansprüche beschreiben vorteilhafte Ausführungsformen der erfindungsgemäßen Vorrichtung.

Die erfindungsgemäße Vorrichtung wird im folgenden anhand der Fig. 1 bis 4 näher erläutert.
Fig. 1 stellt eine Ausführungsform der erfindungsgemäßen Vorrichtung dar.
Fig. 2 zeigt eine Ausführungsform des Resektatextraktors.
Fig. 3 zeigt eine weitere Ausführungsform des Resektatextraktors.
Fig. 4 stellt eine vorteilhafte Ausführungsform des Anschlußstücks dar.

Die erfindungsgemäße Vorrichtung besteht im wesentlichen aus drei Komponenten: einer Rektoskop-Hülse 1, einem Anschlußstück 2 und einem Resektatextraktor 3.

Als Rektoskop-Hülse 1 kann ein Rohr verwendet werden, dessen Durchmesser an den Durchmesser des Anus angepaßt ist. Die Länge der Rektoskop-Hülse ohne Endstück 4 wird im allgemeinen entsprechend der Länge des Rektums (ca. 15 bis 20 cm) gewählt. Um eine Traumatisierung der Rektuminnenwand beim Einführen zu vermeiden, wird das distale Ende der Rektoskop-Hülse vorzugsweise in Form eines Wulstes ausgebildet. Aus dem selben Grund erscheint eine rotationssymmetrische Ausführungsform der Rektoskop-Hülse besser geeignet als die bekannten Rektoskop-Hülsen. Jedoch kann die Rektoskop-Hülse auch in der bekannten Weise distal abgeschrägt sein.

Das proximale Ende der Rektoskop-Hülse wird durch das rohrförmige Endstück 4 gebildet. Der Außendurchmesser des Endstücks ist vorzugsweise größer als der Außendurchmesser der eigentlichen Rektoskop-Hülse, um mehr Platz für die Bohrung, die den Resektatextraktor 3 aufnimmt, für weitere Bohrungen und für die durch die Bohrungen einzuführenden Geräte (Faßzangen, Sichtgeräte etc.) zu schaffen. Eine der weiteren Bohrungen kann in bekannter Weise eine optische Kontrolleinrichtung aufnehmen.

Vorzugsweise wird der Gaseinlaßstutzen 18 am Endstück 4 der Rektoskop-Hülse 1 vorgesehen; hierdurch wird das Ansetzen und Abnehmen des Anschlußstücks erleichtert, denn es hängt in diesem Fall nicht an der Insufflationsleitung. Besonders bevorzugt wird eine solche Ausführungsform, bei der der Gaseinlaßstutzen 18 als Handgriff, etwa in Form einer geriffelten Hülse ausgebildet ist. Ein solcher Handgriff läßt sich während der Operation bei Bedarf auch z. B. über einen einstellbaren Gelenkarm fest mit dem Operationstisch verbinden.

Mit dem Anschlußstück 2 läßt sich die Rektoskop-Hülse 1 an ihrem Endstück 4 dicht verschließen. Ein dichter Verschluß ist erforderlich, um zu verhindern, daß das während der Operation eingeleitete Gas (Kohlendioxid) unkontrolliert entweicht.

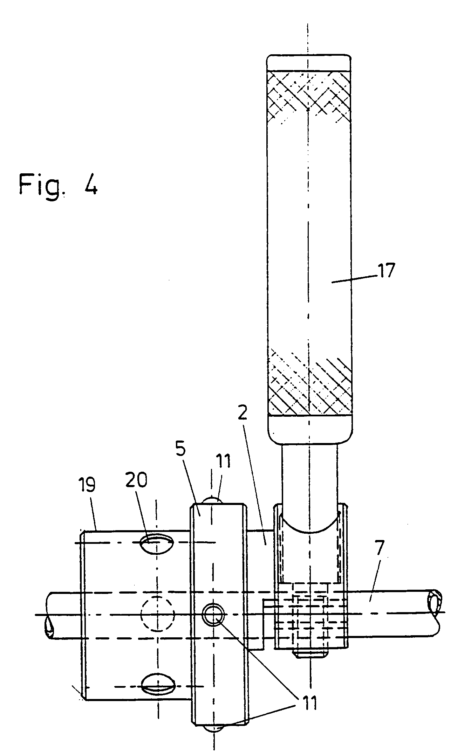

Das Anschlußstück 2 besteht vorzugsweise aus einem Zylinder 5 und einer U-förmigen Klemmvorrichtung 6. Der Zylinder 5 ist vorzugsweise axial durchbohrt. Die Klemmvorrichtung 6 umgreift die Bohrung. Um das Anschlußstück 2 leicht und ohne Verkanten auf das Endstück 4 der Rektoskop-Hülse 1 aufsetzen zu können, ist auf der distalen Seite des Zylinders 5 axialsymmetrisch ein Führungsrohr 19 angebracht. Eine besonders bevorzugte Ausführung dieses Führungsrohrs besteht darin, daß seine Länge so gewählt ist, daß es durch das Endstück 4 bis in den rohrförmigen Bereich der Rektoskop-Hülse reicht. Damit kann ein Verkanten des Anschlußstücks 2 vermieden werden. Fig. 4 zeigt eine solche Ausführungsform des Anschlußstücks 2. Das Führungsrohr 19 enthält Öffnungen, die in der Weise angeordnet sind, daß dann, wenn das Anschlußstück 2 fest mit der Rektoskop-Hülse 1 verbunden ist, der Ausgang des Gaseinleitungsstutzens 18 offen bleibt.

Vorzugsweise werden die Rektoskop-Hülse 1 und das Anschlußstück 2 mit Hilfe eines Bajonettverschlusses miteinander verbunden. In dieser Ausführungsform sind auf der Mantelfläche des Zylinders 5 mindestens zwei, vorzugsweise drei Stifte 11 angebracht, die in entsprechend viele abgewinkelte Aussparungen 9 am Rand des Endstücks 4 eingreifen. Durch die Aussparungen 9 werden federnde Stege 10 am Rand des Endstücks 4 ausgebildet. Die federnden Stege 10 können an einer Stelle eine kleine Ausnehmung von etwa 0,1 mm und daran anschließend einen Anschlag aufweisen, durch die eine eindeutige Verschlußposition der Stifte definiert wird. Dies empfiehlt sich vor allem dann, wenn das Führungsrohr 19 in der in Fig. 4 dargestellten Art ausgebildet ist, damit eine der Öffnungen 20 mit dem Gaseinleitungsstutzen fluchtet.

Den wesentlichen Teil der erfindungsgemäßen Vorrichtung stellt der Resektatextraktor dar. Der Resektatextraktor hat die Aufgabe, die Faßzange, mit der das Resektat ergriffen wird, zu führen und an die richtige Stelle zu positionieren. Er wird so weit anorektal vorgeschoben, bis sein distales Ende aus dem Ende des verbleibenden Darmabschnitts hervortritt. Erfindungsgemäß wird dieses Ende so ausgeformt, daß beim Vorschieben durch das Rektum eine Traumatisierung, insbesondere Läsionen oder Perforationen der Darmwand, vermieden werden können.

Wie erwähnt, wird bei den bekannten Vorrichtungen die Faßzange ohne einen Resektatextraktor durch eine Öffnung des Anschlußstücks in die Rektoskop-Hülse und durch diese hindurch in den Darm geschoben. Das Ende einer Faßzange kann nur in begrenztem Umfang atraumatisch ausgestaltet werden, denn sie muß sich ohne wesentliches Spiel in der Weise durch eine Öffnung im Anschlußstück vorschieben lassen, daß die Öffnung abgedichtet wird. Ferner müssen die beweglichen Teile der Faßzange am distalen Ende frei bleiben, so daß es einer erheblichen Geschicklichkeit des Operateurs bedarf, ein solches Instrument ohne Verletzungen der Darmwand an den gewünschten Ort vorzuschieben.

Mit Hilfe des erfindungsgemäßen Resektatextraktors werden diese Schwierigkeiten überwunden. Der Resektatextraktor läßt sich in der Weise gestalten, daß die Flächenpressung zwischen Instrumenten- und Darmwand, die letztlich bei zu hohen Werten zur Perforation des Darmes führt, auf unkritische Werte reduziert werden und das sichere Übergleiten von Darmunregelmäßigkeiten erleichtert wird. Die Belastung der Darmwand bei diesem Vorgang läßt sich weiterhin dadurch reduzieren, daß der CO₂-Druck in der Rektoskop-Hülse über den Gasanschlußstutzen 18 bis etwas über den Pneumoperitoneumdruck erhöht wird, um das Rektum zu weiten und teilweise von der Außenfläche des Protektorabschnitts abzuheben.

Erst wenn der Resektatextraktor wie beschrieben positioniert ist, wird die Faßzange nach Entfernen des Verschlußstopfens 21 durch den Rohrabschnitt und den Protektorabschnitt des Resektatextraktors bis ins Abdomen vorgeschoben und mit ihr das Resektat ergriffen.

Auf diese Weise ist ein Traumatisierung des Rektums durch die Faßzange völlig ausgeschlossen.

Wenn der Operateur mit der Faßzange das Resektat ergriffen hat, werden sowohl die Faßzange als auch der Resektatextraktor zurückgezogen. Das Resektat wird wie üblich aus der Rektoskop-Hülse entfernt, indem das Anschlußstück abgenommen wird.

Erfindungsgemäß werden zwei Ausführungsformen des Resektatextraktors vorgeschlagen. Bei beiden Ausführungsformen besteht der Resektatextraktor aus einem Rohrabschnitt 7 und einem verdickten, durchbohrten Abschnitt am distalen Ende.

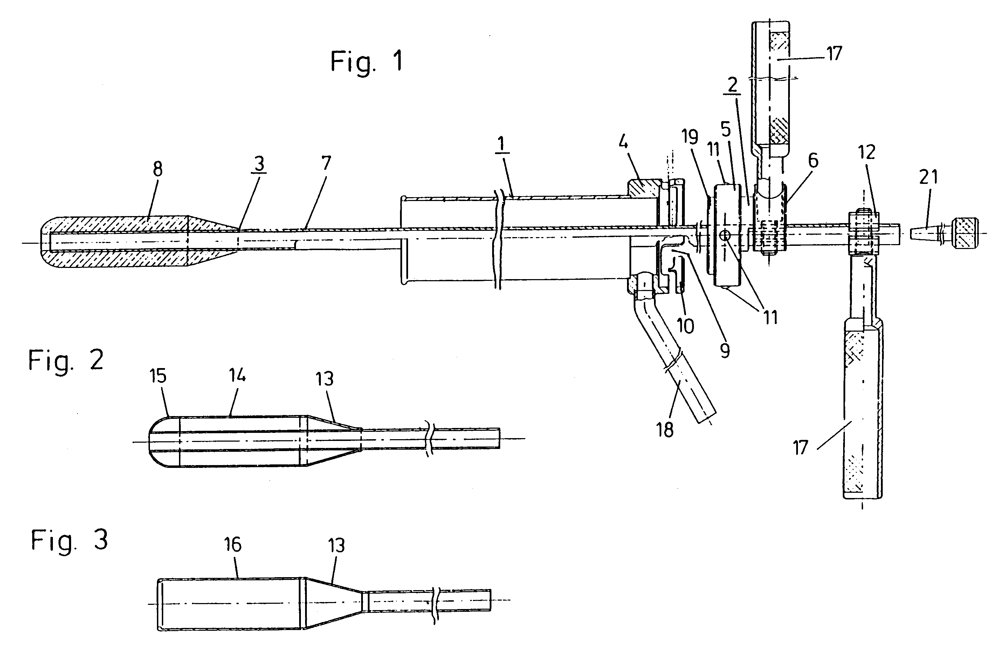

Bei der ersten Ausführungsform, die in Fig. 2 dargestellt ist, ist der Abschnitt am distalen Ende keulenförmig erweitert. Der Rohrabschnitt geht über einen Übergangsbereich 13 in einen im wesentlichen zylindrischen Bereich 14 mit größerem Außendurchmesser über, der durch einen kalottenförmigen, z. B. halbkugeligen Protektorbereich 15 mit einer Öffnung am distalen Ende abgeschlossen wird. Diese Ausführungsform ist in besonderer Weise in Bezug auf eine möglichst geringe Flächenpressung optimiert. Die Länge des Übergangsbereichs 13 und des zylindrischen Bereichs 14 sowie der Öffnungswinkel des Übergangsbereichs werden so gewählt, daß ein möglichst strömungsgünstiger Körper entsteht. Die Länge der drei Bereiche 13, 14, 15 soll kürzer sein als die Länge der Rektoskop-Hülse, so daß das Resektat in deren Bereich zurückgezogen werden kann. Es ist gegebenenfalls auch möglich, auf den zylindrischen Bereich 14 zu verzichten oder den Protektorbereich als Paraboloid auszubilden.

Die zweite Ausführungsform, die in Fig. 3 dargestellt ist, wird für die Resektion von infektiösem oder entartetem Gewebe vorgeschlagen. Bei dieser Ausführungsform kann der Rohrabschnitt 7 am distalen Ende des Resektatextraktors trichterförmig erweitert sein. An die trichterförmige Erweiterung im Übergangsbereich 13 kann sich ein zylindrischer Bereich 16 anschließen, in dem sowohl der Außen- wie auch der Innendurchmesser gegenüber dem Rohrabschnitt 7 vergrößert ist. Der distale Rand dieses Resektatextraktors wird durch einen verdickten und eingezogenen Wulst atraumatisch gestaltet. Bei dieser Ausführungsform ist es möglich, das Resektat mit Hilfe der Faßzange in den Innenraum des zylindrischen Bereichs 16 zu ziehen, so daß die Rektuminnenwand beim Zurückziehen des Resektatextraktors nicht mit dem zu entfernenden Gewebe in Kontakt kommt.

Die Bedienung der erfindungsgemäßen Vorrichtung wird wesentlich erleichtert, wenn auf den außerhalb der Rektoskop-Hülse liegenden Rohrabschnitt 7 des Resektatextraktors 3 eine verschiebbare Klemmvorrichtung 12 aufgeschoben wird. Vorzugsweise sind sowohl die am Anschlußstück 2 angebrachte Klemmvorrichtung 6 und die auf dem Rohrabschnitt 7 verschiebbare Klemmvorrichtung 12 in derselben Weise aufgebaut.

Eine besonders einfache Bedienung wird mit solchen Klemmvorrichtungen erzielt, bei denen ein U-förmiges Teil den Rohrabschnitt 7 umschließt, wobei die Schenkel dieses Teils durchbohrt sind. In eine der Bohrungen ist ein Gewinde eingeschnitten. Eine Schraube greift durch eine der Bohrungen in die mit dem Gewinde versehene Bohrung. Der Schaft dieser Schraube ist als Handgriff ausgebildet. Besonders bevorzugt wird ein Handgriff in Form einer Hülse, dessen Außenseite geriffelt ist. Hierdurch kann in beträchtlichem Maß Gewicht eingespart werden. Bei einer solchen Klemmvorrichtung ist eine Einhandbedienung möglich.

Das proximale Ende des Resektatextraktors kann dann, wenn die Faßzange nicht eingeschoben ist, durch einen Stopfen 21 mit Silikontülle verschlossen werden. Andernfalls wird der Resektatextraktor durch die Faßzange selbst oder durch darauf angebrachte Dichtungselemente abgedichtet.

Als Werkstoff für die erfindungsgemäße Vorrichtung eignet sich vor allem ein Edelstahl. In diesem Fall kann die gesamte Vorrichtung einfach sterilisiert werden, wobei für gegebenenfalls notwendige Dichtungshilfsmittel Einmalartikel verwendet werden. Der Protektorabschnitt des Resektatextraktors kann jedoch auch aus Polytetrafluorethylen (PTFE) bestehen, wie in Fig. 1 dargestellt. PTFE ist gewebefreundlich, wasserabstoßend und kann ebenfalls bei hohen Temperaturen sterilisiert werden. Es besteht jedoch auch die Möglichkeit, die erfindungsgemäße Vorrichtung oder einzelne Teile davon wie z. B. den Resektatextraktor in einem Spritzgießverfahren aus einem Kunststoff herzustellen und nach Gebrauch zu vernichten.

## Patentansprüche

1. Vorrichtung für transanale Resektatextraktionen, mit
a) einer Rektoskop-Hülse (1) mit einem proximal angeordneten rohrförmigen Endstück (4),
b) einem Anschlußstück (2), durch das sich das rohrförmige Endstück (4) dicht verschließen läßt und das eine zur Achse der Rektoskop-Hülse (1) und zur Achse des rohrförmigen Endstücks (4) parallele Bohrung aufweist,
gekennzeichnet durch
c) einen Resektatextraktor (3) zur Aufnahme, Führung und Positionierung einer Faßzange, mit einem Rohrabschnitt (7) und einem distal am Rohrabschnitt (7) angebrachten Protektorabschnitt (8), wobei die Länge des Rohrabschnittes (7) die Länge der Rektoskop-Hülse (1) übertrifft, sodaß die Faßzange anorektal an die jeweilige Stelle positionierbar ist, wobei der Rohrabschnitt (7) sich mit geringem Spiel durch die Bohrung des Anschlußstücks (2) führen läßt und der Protektorabschnitt (8) durchbohrt ist und einen Außendurchmesser im Bereich zwischen dem Innendurchmesser der Rektoskop-Hülse (1) und dem Außendurchmesser des Rohrabschnitts (7) aufweist.

2. Vorrichtung nach Anspruch 1, dadurch gekennzeichnet, daß das rohrförmige Endstück mindestens zwei abgewinkelte Aussparungen (9) enthält, durch die am Rand des Endstücks (4) federnde, einseitig gehaltene Stege (10) ausgebildet sind, und das Anschlußstück aus einem axial durchbohrten Zylinder (5) besteht, auf dessen Mantelfläche mindestens zwei in die abgewinkelten Aussparungen (9) eingreifende Stifte (11) angeordnet sind.

3. Vorrichtung nach Anspruch 1 oder 2, dadurch gekennzeichnet, daß an dem Anschlußstück (2) auf der proximalen Seite eine U-förmige Klemmvorrichtung (6) angebracht ist, die die Bohrung umfaßt.

4. Vorrichtung nach Anspruch 1 oder 3, dadurch gekennzeichnet, daß auf dem Rohrabschnitt (7) des Resektatextraktors (3) eine frei verschiebbare U-förmige Klemmvorrichtung (12) angebracht ist.

5. Vorrichtung nach Anspruch 1, dadurch gekennzeichnet, daß der Protektorabschnitt (8) über einen kegelförmigen Übergangsbereich (13) und einen Zylinderbereich (14) in einen halbkugelig ausgeformten Protektorbereich (15) ausläuft, wobei der Durchmesser der Durchbohrung des Protektorabschnitts (8) dem Innendurchmesser des Rohrabschnitts (7) entspricht.

6. Vorrichtung nach Anspruch 1, dadurch gekennzeichnet, daß der Protektorabschnitt (7) über einen kegelförmigen Übergangsbereich (13) in einen stirnseitig offenen Zylinderbereich (16) übergeht, wobei der stirnseitig offene Zylinderbereich (16) einen zylindrischen Innenraum umschließt.

7. Vorrichtung nach Anspruch 3 oder 4, dadurch gekennzeichnet, daß daß die U-förmige Klemmvorrichtung (6, 12)
- in den Schenkeln des U zwei fluchtende Bohrungen aufweist,
- in eine der Bohrungen ein Gewinde eingeschnitten ist,
- eine Schraube durch eine der Bohrungen in die mit dem Gewinde versehene Bohrung eingreift und
- der Schaft der Schraube als Handgriff (17) ausgebildet ist.

8. Vorrichtung nach Anspruch 7, dadurch gekennzeichnet, daß der Handgriff (17) eine außen geriffelte Hülse darstellt.

9. Vorrichtung nach Anspruch 1 , dadurch gekennzeichnet, daß in das rohrförmige Endstück (4) der Rektoskophülse (1) ein Gaseinleitungsstutzen (18) integriert ist.

10. Vorrichtung nach Anspruch 9, dadurch gekennzeichnet, daß der Gaseinleitungsstutzen (18) als Handgriff ausgebildet ist.

## Claims

1. Device for transanal resectate extractions, having
a) a rectoscope sleeve (1) provided with a proximally disposed tubular end-piece (4),
b) a connecting-piece (2), by means of which the tubular end-piece (4) can be tightly sealed, and which includes a bore, which extends parallel to the axis of the rectoscope sleeve (1) and the axis of the tubular end-piece (4),
characterised by
c) a resectate extractor (3) for picking-up, guiding and positioning a gripping means, said extractor having a tubular portion (7) and a protector portion (8), which is mounted at the distal end of the tubular portion (7), the length of the tubular portion (7) exceeding the length of the rectoscope sleeve (1), so that the gripping means is positionable anorectally at the particular location, the tubular portion (7) being guidable through the bore of the connecting-piece (2) with little clearance, and the protector portion (8) having a through-bore and having an external diameter in the range of between the internal diameter of the rectoscope sleeve (1) and the external diameter of the tubular portion (7).

2. Device according to claim 1, characterised in that the tubular end-piece includes at least two angular recesses (9), through which unilaterally retained webs (10) extend, said webs being resilient at the edge of the end-piece (4), and the connecting-piece comprises an axially pierced cylinder (5), on the surface of which are disposed at least two pins (11), which engage in the angular recesses (9).

3. Device according to claim 1 or 2, characterised in that a U-shaped clamping device (6), which surrounds the bore, is mounted on the proximal side of the connecting-piece (2).

4. Device according to claim 1 or 3, characterised in that a freely displaceable U-shaped clamping device (12) is mounted on the tublar portion (7) of the resectate extractor (3).

5. Device according to claim 1, characterised in that the protector portion (8) extends into a domed protector region (15) via a conical transitional region (13) and a cylindrical region (14), the diameter of the through-bore of the protector portion (8) corresponding to the internal diameter of the tubular portion (7).

6. Device according to claim 1, characterised in that the protector portion (7) extends via a conical transitional region (13) into a cylindrical region (16), which is open at its end face, the cylindrical region (16) which is open at its end face surrounding a cylindrical interior.

7. Device according to claim 3 or 4, characterised in that the U-shaped clamping device (6, 12)
- as two aligned bores in the legs of the "U",
- a thread is cut into one of the bores,
- a screw through one of the bores engages in the bore provided with the thread, and
- the shaft of the screw is configured as handle (17).

8. Device according to claim 7, characterised in that the handle (17) constitutes an externally grooved sleeve.

9. Device according to claim 1, characterised in that a gas inlet pipe (18) is incorporated in the tubular end-piece (4) of the rectoscope sleeve (1).

10. Device according to claim 9, characterised in that the gas inlet pipe (18) is configured as the handle.

## Revendications

1. Dispositif pour l'extraction transanale de matières de résection, comportant :
a) un manchon rectoscopique (1) avec une pièce terminale proximale(4) en forme de tube,
b) une pièce de raccordement (2) qui permet de fermer de façon étanche la pièce terminale (4) en forme de tube et qui présente un perçage parallèle à l'axe de la pièce terminale (4) en forme de tube,
caractérisé par :
c) un extracteur de matières de résection (3) qui sert à recevoir, guider et mettre en place une pince de préhension, avec une section de tube (7) et une section de protecteur (8) mis sur la partie distale de la section de tube (7), la longueur de la section de tube (7) dépassant la longueur d'un manchon rectoscopique (1), de telle sorte que la pince de préhension puisse être mise en place de façon anorectale à l'endroit correspondant,
la section de tube (7) pouvant être guidée avec un faible jeu à travers l'alésage de la pièce de raccordement (2), tandis que la section de protecteur (8) est percée et présente un diamètre extérieur se situant dans la zone comprise entre le diamètre intérieur du manchon recto-scopique (1) et le diamètre extérieur de la section de tube (7).

2. Dispositif selon la revendication 1,
caractérisé en ce que
la pièce terminale en forme de tube contient au moins deux évidements (9) formant un angle, évidements grâce auxquels sont constituées des entretoises (10) montées de façon élastique sur le bord de la pièce terminale (4) et maintenues d'un côté, la pièce de raccordement consistant en un cylindre (5) percé axialement, sur la surface enveloppe duquel sont disposés au moins deux axes (11) venant en prise dans les évidements (9) formant un angle.

3. Dispositif selon la revendication 1 ou 2,
caractérisé en ce que,
on met sur la pièce de raccordement (2), du côté proximal, un dispositif de serrage (6) en forme de U qui entoure l'alésage.

4. Dispositif selon la revendication 3,
caractérisé en ce que,
on met sur la section de tube (7) de l'extracteur des matières de résection (3), un dispositif de serrage (12) en forme de U qui peut coulisser librement.

5. Dispositif selon la revendication 1,
caractérisé en ce que,
la section de protecteur (8) s'étend, via une zone de transition (13) en forme de cône et une zone cylindrique (14), jusqu'à une zone du protecteur (15) ayant une forme hémisphérique, le diamètre du perçage de la section de protecteur (8) correspondant au diamètre intérieur de la section de tube (7).

6. Dispositif selon la revendication 1,
caractérisé en ce que,
la section de protecteur (8) se transforme, via une zone de transition (13) en forme de cône, en une zone cylindrique (16) ouverte du côté frontal, la zone cylindrique (16) ouverte du côté frontal entourant un espace intérieur cylindrique.

7. Dispositif selon la revendication 3 ou 4,
caractérisé en ce que,
le dispositif de serrage en forme de U (6, 12) est tel que :
- les branches du U présentent deux perçages en alignement,
- un filetage est taillé dans l'un des perçages,
- une vis vient en prise, à travers l'un des perçages dans le perçage qui est pourvu du filetage,
- la tige de la vis est constituée en forme de poignée (17).

8. Dispositif selon la revendication 7,
caractérisé en ce que,
la poignée (17) constitue un manchon strié extérieurement.

9. Dispositif selon la revendication 8,
caractérisé en ce que,
on intègre dans la pièce terminale (4) de forme tubulaire du manchon rectoscopique (1), un ajutage d'introduction de gaz (18).

10. Dispositif selon la revendication (9),
caractérisé en ce que
l'ajutage d'introduction de gaz (18) est constitué sous la forme d'une poignée.
